# EUROPÄISCHE PATENTSCHRIFT

(11) **EP 0 519 297 B1**
(45) Veröffentlichungstag und Bekanntmachung des Hinweises auf die Patenterteilung: **20.09.1995**
(21) Anmeldenummer: 92109651.7
(22) Anmeldetag: 09.06.1992
(51) Int. Cl.: C07C 233/27, C08F 20/58, G03F 7/039

(54) **Strahlungsempfindliches Gemisch, das als Bindemittel neue Polymere mit Einheiten aus Amiden von alpha,beta-ungesättigten Carbonsäuren enthält**
Radiation-sensitive composition comprising as binders novel polymers with units derived from amides of alpha,beta-unsaturated carboxylic acids
Composition sensible aux rayonnements comprenant nouveaux polymères à base d'amides des acides carboxyliques alpha, beta-insaturés comme liant

(30) Priorität: 19.06.1991 DE 4120172
(43) Veröffentlichungstag der Anmeldung: 23.12.1992
(73) Patentinhaber: HOECHST AKTIENGESELLSCHAFT, 65926 Frankfurt am Main (DE)
(72) Erfinder: Przybilla, Klaus-Jürgen, Dr., W-6000 Frankfurt am Main 1 (DE); Pawlowski, Georg, Dr., W-6200 Wiesbaden (DE); Röschert, Horst, Dr., W-6531 Ober Hilbersheim (DE)

(56) Entgegenhaltungen:
- EP-A- 0 212 440
- DE-A- 3 825 738
- US-A- 4 908 381
- PATENT ABSTRACTS OF JAPAN, vol. 13, no. 547 (P-971)(3895), 7. Dezember 1989 & JP-A-12 27 143

## Beschreibung

Die Erfindung betrifft ein strahlungsempfindliches Gemisch, das ein polymeres Bindemittel mit säurespaltbaren Seitengruppen und eine bei Bestrahlung eine starke Säure bildende Verbindung enthält.

Die Erfindung betrifft daneben neue Amide von α,β-ungesättigten Carbonsäuren, mit denen die als Bindemittel verwendeten Polymere aufgebaut werden.

Strahlungsempfindliche Gemische sind an sich bekannt. Kommerziell werden besonders positiv arbeitende Gemische als Resistmaterial verwendet, die neben o-Chinondiazid ein in wäßrig-alkalischer Lösung lösliches Bindemittel, wie Poly(4-hydroxy-styrol) oder ein Novolak, enthalten. Die Empfindlichkeit dieser Systeme gegenüber Strahlung, besonders kurzwelliger Strahlung, ist jedoch teilweise unbefriedigend. Durch die hohe intrinsische Absorption im UV-2-Bereich (220-300 nm) sind Novolake als Bindemittel in einem Einlagen-Resistmaterial für die Deep-UV-Lithographie (220-330 nm) ungeeignet. Poly(hydroxystyrol) weist demgegenüber günstigere Absorptionseigenschaften im UV-Bereich auf und zeichnet sich auch durch einen höheren Wärmestand aus. Jedoch ist dieses Polymer nur durch aufwendige mehrstufige Synthesen zugänglich, und seine lithographischen Eigenschaften sind wegen seiner unausgewogenen Hydrophilie/Hydrophobie-Bilanz sowohl in 3-Komponenten- als auch in 2-Komponenten-Systemen nicht unproblematisch. Daher besteht ein Bedarf an Bindemitteln für hochauflösende, hochempfindliche Resistmaterialien mit guter Ätzresistenz, guter Transparenz und hohem Wärmestand für die UV-2-Lithographie, die wäßrigalkalisch entwickelbar sind.

Bekannt ist es auch, die Strahlungsempfindlichkeit von strahlungsempfindlichen Gemischen durch den Zusatz einer Verbindung zu erhöhen, die unter Einwirkung von Strahlung eine Säure freisetzt, die dann Sekundärreaktionen katalysiert. Solche Verbindungen sind z. B. Diazonium-, Phosphonium-, Sulfonium- und Iodoniumsalze, Nitrobenzylester, phenolische Methansulfonate, Diazo- und Halogenverbindungen. Die Verwendung der Oniumsalze als photochemische Säurebildner in Resistmaterialien ist z. B. aus der US-A 4 491 628 bekannt. Einen Überblick über die Anwendungen von Oniumsalzen in Resistmaterialien gibt Crivello in Org. Coatings and Appl. Polym. Sci., 48, p. 65-69 (1985).

Strahlungsempfindliche Gemische von Polymeren mit säurelabilen Seitengruppen und photochemischen Säurebildnern sind aus US-A 4 491 628 und FR-A 2 570 844 (≈ US-A 4 689 288 und 4 770 977) bekannt. Konkret offenbart sind in den beiden genannten Schriften allein Polymere aus para-substituiertem Styrol oder α-Alkylstyrol. Als säurelabile para-Substituenten sind u.a. tert.-Butoxycarbonyloxy- und Trialkylsilanyloxy-Gruppen aufgeführt. Diese polymeren Bindemittel sind hydrophob und werden erst nach der Belichtung alkalilöslich.

Copolymere mit säurelabilen Gruppen, die über ein phenolisches Sauerstoffatom gebunden sind, beispielsweise Copolymere aus p-Hydroxystyrol und tert.-butoxycarbonyloxystyrol, sind aus J.Poly. Sci., Part A, Polym. Chem. Ed., Vol. 24, 2971-2980 (1986) bekannt.

Aufgabe der vorliegenden Erfindung ist es, ein neues, im kurzwelligen UV-Bereich hoch strahlungsempfindliches Gemisch für die Herstellung von Reliefstrukturen bereitzustellen, das sich mit wäßrig-alkalischen Lösungen entwickeln läßt.

Gelöst wird die Aufgabe durch ein strahlungsempfindliches Gemisch, das ein polymeres Bindemittel mit säurespaltbaren Seitengruppen und eine bei Bestrahlung eine starke Säure bildende Verbindung enthält. Das strahlungsenpfindliche Gemisch ist dadurch gekennzeichnet, daß es neue Polymere mit Einheiten der allgemeinen Formel (I) 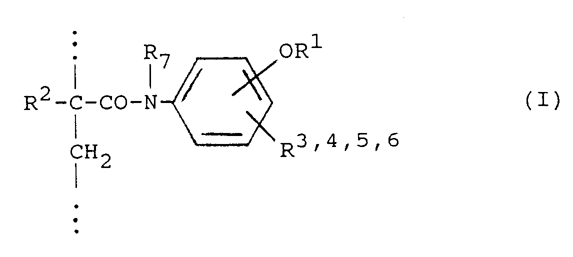
enthält, worin
- R¹: eine Benzyl-, Trialkylsilanyl-, Alkoxycarbonyl-, Tetrahydropyranyl- oder Tetrahydrofuranyl-Gruppe und
- R²: (C₁-C₄)Alkyl, Halogen(C₁-C₄)alkyl oder Wasserstoff bedeuten,
- R^{3,4,5,6}: voneinander unabhängig einen gegebenenfalls halogen-substituierten aliphatischen, araliphatischen oder aromatischen Rest mit 1 bis 20 Kohlenstoffatomen, ein Halogenatom oder Wasserstoff darstellen und
- R⁷: (C₁-C₄)Alkyl oder Wasserstoff ist.

Das erfindungsgemäße Gemisch zeigt im Vergleich zu den bekannten Gemischen mit Polystyrol, Novolaken oder Polymeren mit Einheiten aus Estern von α,β-ungesättigten Carbonsäuren als Bindemittel folgende Vorteile:
a) die monomeren Amide der α,β-ungesättigten Carbonsäuren lassen sich durch die höhere Nucleophilie der an einen aromatischen Ring gebundenen Aminogruppen selektiv synthetisieren,
b) die Synthese der monomeren Amide der α,β-ungesättigten Carbonsäuren erfolgt ohne organisches Lösemittel in Wasser, was ein beachtlicher Verfahrensvorzug ist,
c) der Wärmestand von damit hergestellten Polymeren ist durch die Natur der Amid-Bindung und die daraus resultierenden Wasserstoffbrückenbindungen wesentlich höher als der von Polymeren aus entsprechenden Estern,
d) leichte Zugänglichkeit der geschützten Monomere,
e) Stabilität der ungeschützten Monomere,
f) Homo- und Copolymerisierbarkeit zu hohen Molekulargewichten der geschützten sowie der ungeschützten Monomere,
g) gute Ätzresistenz,
h) ausgewogene Hydrophilie/Hydrophobie-Bilanz und
i) gute Haftungseigenschaften.

Polymere mit Einheiten der allgemeinen Formel I sind prinzipiell auf zwei verschiedenen Wegen herstellbar.

Zum einen können sogenannte "geschützte" Monomere der allgemeinen Formel II 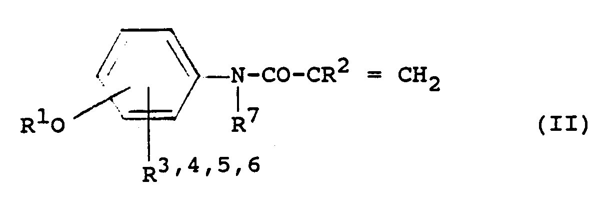
worin R¹ bis R⁷ die oben angegebene Bedeutung haben, entweder allein oder mit anderen Monomeren polymerisiert werden. Diese Monomere sind neu und Teil der vorliegenden Erfindung. Beispiele für Monomere der allgemeinen Formel II sind mit säurelabilen Gruppen R¹ gebildete Derivate des N-(2-, 3- und 4-Hydroxy-phenyl)-(meth)acrylamids. Als säurespaltbare Gruppe R¹ ist besonders Benzyl, Trialkylsilanyl, tert.-Butoxycarbonyl, Isopropoxycarbonyl, Benzyloxycarbonyl, Pentyloxycarbonyl, Tetrahydropyranyl oder Tetrahydrofuranyl geeignet. Besonders bevorzugt sind Monomere mit R² = H oder Methyl und R³ = R⁴ = R⁵= R⁶ = H. Besonders bevorzugte Monomere sind N-(4-tert.-Butoxycarbonyloxy-phenyl)-(meth)acrylamid, N-(3-tert.-Butoxycarbonyloxy-phenyl)-(meth)acrylamid und N-(2-tert.-butoxycarbonyloxy-phenyl)-(meth)acrylamid.

Zum anderen können die N-(Hydroxyphenyl)-(methacryl)amide zu Polymeren mit hohen Molekulargewichten polymerisiert werden, da diese in monomerer Form stabil sind. Diese Polymere können dann in einem Folgeschritt derivatisiert werden, um die Gruppen R¹ einzuführen. Diese Verfahrensweise ist jedoch nicht bevorzugt, da sich die phenolischen Hydroxygruppen in den Polymeren nicht immer in reproduzierbarer Weise derivatisieren lassen. Zugleich ist es bei dem zweistufigen Verfahren unvermeidlich, daß Reste der bei der Derivatisierungsreaktion verwendeten Base im Produkt verbleiben.

4-Hydroxystyrol ist dagegen in freier Form nicht beständig. Es läßt sich zudem nur in einer 4-Stufen-Synthese mit schlechten Ausbeuten herstellen. Deshalb kann als Ausgangsmaterial für die Polymerisation nur ein geschütztes 4-Hydroxystyrol, wie 4-(tert.-Butoxycarbonyloxy)-styrol, verwendet werden. Letzteres wird über eine Wittig-Reaktion aus 4-Hydroxy-benzaldehyd hergestellt (US-A 4 491 628). Alternativ dazu ist Poly(4-hydroxy-styrol) mit geeigneten Schutzgruppen umgesetzt worden. Dieses Herstellungsverfahren hat jedoch den gravierenden Nachteil, daß dadurch das Bindemittel mit Metallionen und mit Base kontaminiert wird. Das Vorhandensein von Basen ist für photochemisch verstärkt arbeitende Systeme nachteilig. Eine hohe Metallionenkontamination ist für die Herstellung von Halbleitern sogar inakzeptabel. Nach diesem Verfahren hergestellte Bindemittel zeigen häufig nicht reproduzierbare lithographische Eigenschaften.

Diese Nachteile sind mit den erfindungsgemäßen Polymeren und dem damit hergestellten erfindungsgemäßen strahlungsempfindlichen Gemisch nicht mehr verbunden.

Die erfindungsgemäßen polymeren Bindemittel können sowohl Homopolymere, die ausschließlich Einheiten der allgemeinen Formel I enthalten, als auch Co- und Terpolymere sein. In den Co- und Terpolymeren sind - neben den Einheiten der allgemeinen Formel I - bevorzugt Einheiten, abgeleitet von "ungeschützten" und/oder säurestabil geschützten Monomeren, der allgemeinen Formel II vorhanden (R¹ ist in diesen Monomeren Wasserstoff bzw. ein durch Säure nicht abspaltbarer Rest). In den Co- und Terpolymeren können jedoch auch Einheiten vorkommen, die von anderen üblichen Vinylmonomeren abgeleitet sind. Als solche weiteren Monomere können besonders Ester oder Amide der (Meth)acrylsäure, wie Hydroxyethylmethacrylat (HEMA), aber auch Methacrylsäure selbst, Hydroxypropylmethacrylat, Methylmethacrylat, Ethylmethacrylat, Methacrylamid und Hydroxyethylmethacrylamid, fungieren. Die Haftungseigenschaften des Bindemittels lassen sich dadurch modifizieren.

Gemische mit erhöhter Plasmabeständigkeit werden dann erhalten, wenn zur Herstellung von Co- oder Terpolymeren Silicium enthaltende Monomere verwendet werden.

Schließlich sind darüber hinaus auch Copolymere mit Maleimid verwendbar, die in wäßrig alkalischen Lösungen eine erhöhte Löslichkeit zeigen und eine höhere Transparenz im Deep-UV-Bereich aufweisen. Den gleichen Effekt zeigen auch Copolymere mit Styrol, substituiertem Styrol, mit Vinylethern, Vinylestern, Vinylsilanverbindungen oder (Meth)acrylsäureestern.

Die erfindungsgemäßen Polymere enthalten mindestens 10 mol-% an Einheiten der allgemeinen Formel I. Ihr mittleres Molekulargewicht Mₙ liegt zwischen 2.000 und 100.000, vorzugsweise zwischen 5.000 und 40.000 g/mol.

Das Bindemittel mit säurespaltbaren Gruppen ist im erfindungsgemäßen Gemisch im allgemeinen in Mengen von 45 bis 98 Gew.-%, vorzugsweise 90 bis 98 Gew.-%, bezogen auf das Gesamtgewicht der Feststoffe im strahlungsempfindlichen Gemisch, enthalten.

Das erfindungsgemäße Gemisch enthält weiterhin eine photoaktive Verbindung, die bei Bestrahlung eine starke Säure freisetzt, die ihrerseits in dem Bindemittel vorhandene Schutzgruppen abspaltet und dadurch bewirkt, daß die Löslichkeit des Bindemittels und somit auch des Gemisches in wäßrig-alkalischer Lösung stark ansteigt. Das Gemisch ist besonders sensitiv gegenüber UV(220-400 nm)-, Elektronen- und Röntgenstrahlen und eignet sich besonders als Resistmaterial, doch auch zur Herstellung von Druckplatten.

Als Säurebildner kommen im Prinzip alle Verbindungen in Frage, die bei Bestrahlung eine starke Säure bilden. Beispiele besonders geeigneter Säurebildner sind Sulfoniumsalze der allgemeinen Formel [(C₆H₅)₃S]⁺X⁻, wobei X besonders für Chlorid, Bromid, Perchlorat, Hexafluorphosphat, Hexafluorarsenat, Hexafluorantimonat, Tetrafluorborat oder ein Sulfonat, wie Methansulfonat, Trifluormethansulfonat, Tosylat oder Hexafluorpropansulfonat, steht. Geeignet sind auch Nitrobenzylester, Bissulfonyl-diazomethane, Pyrogallol-sulfonsäureester, 1-Sulfonyloxy-2-pyridone und Halogenverbindungen. Für die Bestrahlung mit kurzwelligen UV-Strahlen sind jedoch Jodonium- und besonders Sulfoniumsalze bevorzugt.

Der Säurebildner ist in dem Gemisch in einer Menge von 1 bis 40 Gew.-%, vorzugsweise 3 bis 10 Gew.-%, bezogen auf das Gesamtgewicht der Feststoffe in dem strahlungsempfindlichen Gemisch, vorhanden.

Das erfindungsgemäße strahlungsempfindliche Gemisch kann zusätzlich noch weitere übliche Hilfs- und Zusatzstoffe enthalten, wie Haftvermittler, Netzmittel, Farbstoffe und Weichmacher.

Gegebenenfalls können auch Sensibilisatoren in geringen Mengen zugesetzt werden, um den Säurebildner für Strahlung im längerwelligen UV- bis hin zum sichtbaren Bereich zu sensibilisieren. Polycyclische Aromaten wie Pyren und Perylen sind hierfür bevorzugt, jedoch können auch Farbstoffe, die als Sensibilisatoren wirken, verwendet werden.

Ferner betrifft die Erfindung ein Aufzeichnungsmaterial mit einem Träger und einer Schicht, die das erfindungsgemäße strahlungsempfindliche Gemisch enthält, sowie ein Verfahren zur Herstellung von Reliefstrukturen oder zur Strukturierung von Wafern.

Um das erfindungsgemäße Gemisch auf ein Trägermaterial auftragen zu können, wird es zweckmäßig in einem organischen Lösemittel gelöst, wobei der Feststoffgehalt im allgemeinen im Bereich zwischen 5 und 40 Gew.-% liegt. Als Lösemittel kommen bevorzugt aliphatische Ketone, Ether und Ester sowie beliebige Mischungen davon in Frage. Besonders bevorzugt sind Alkylenglykol-monoalkylether, wie beispielsweise Ethylcellosolve, Ethylenglykol-mono-butylether, Methylcellosolve und 1-Methoxy-2-propanol, Alkylenglykolalkylether-ester, wie z. B. Methylcellosolve-acetat, Ethylcellosolve-acetat, Propylenglykol-methylether-acetat und Propylenglykolethylether-acetat, Ketone, wie beispielsweise Cyclohexanon, Cyclopentanon und Butanon, sowie Acetate, wie Butylacetat, und Aromaten, wie Toluol und Xylol. Die Auswahl der entsprechenden Lösemittel bzw. deren Mischungen richtet sich nach der Wahl des jeweiligen phenolischen Polymers und der photoempfindlichen Komponente.

Bei dem erfindungsgemäßen Verfahren zur Herstellung von Reliefmustern wird eine strahlungsempfindliche Aufzeichnungsschicht, die im wesentlichen aus dem erfindungsgemäßen strahlungsempfindlichen Gemisch besteht, bildmäßig in solcher Dosis bestrahlt, daß die Löslichkeit der belichteten Bereiche in wäßrig-alkalischen Lösemitteln zunimmt und diese bestrahlten Bereiche dann selektiv mit dem alkalischen Entwickler entfernt werden können. Man enthält auf diese Weise ein positives Bild.

Ebenso ist auch eine Entwicklung mit organischen Lösemitteln wie Toluol oder Anisol möglich. Die unbelichteten Bereiche zeigen eine größere Lipophilie und werden daher abgetragen. Man erhält ein negatives Bild.

Das erfindungsgemäße strahlungsempfindliche Gemisch arbeitet je nach Wahl der Entwicklungsverfahren positiv oder negativ. Es eignet sich besonders als Photoresist zur Herstellung von Reliefstrukturen für Halbleiterbauelemente.

Die das erfindungsgemäße strahlungsempfindliche Gemisch enthaltenden Photoresistlösungen werden im allgemeinen in Schichten von 0,1 bis 5 µm, vorzugsweise 0,5 bis 1,5 µm auf geeignete Substrate, beispielsweise oberflächlich oxidierte Silicium-Wafer durch Aufschleudern (spin coating) aufgetragen, getrocknet (z. B. bei Temperaturen zwischen 70 und 130 °C) und mit einer geeigneten Lichtquelle durch eine Photomaske bildmäßig belichtet. Als Lichtquellen eignen sich insbesondere kurzwellige UV-Strahlen (deep UV) mit Wellenlängen zwischen 200 und 400 nm. Besonders geeignete Lichtquellen sind Excimer-Laser.

Nach dem bildmäßigen Belichten wird gegebenenfalls nach kurzem Ausheizen (post-bake) bei Temperaturen bis zu 150 °C mit üblichen wäßrig-alkalischen Entwicklerlösungen, im allgemeinen bei pH-Werten zwischen 12 und 14, entwickelt, wobei die belichteten Stellen ausgewaschen werden. Die Auflösung liegt im Subhalbmikron-Bereich. Die für das erfindungsgemäße strahlungsempfindliche Gemisch benötigte Belichtungsenergie liegt im allgemeinen zwischen 5 und 200 mJ/cm² bei Schichtdicken von 1 µm.

Die entwickelten Resiststrukturen werden gegebenenfalls nachgehärtet. Dies geschieht im allgemeinen dadurch, daß man die Resiststruktur auf einer hot-plate bis zu einer Temperatur unter der Fließtemperatur erhitzt und anschließend mit UV-Licht einer Xenon-Quecksilberdampflampe (Bereich 200 bis 250 nm) ganzflächig belichtet. Durch diese Nachhärtung werden die Resiststrukturen vernetzt, so daß die Strukturen eine Fließbeständigkeit im allgemeinen bis zu Temperaturen von über 200 °C aufweisen. Die Nachhärtung kann auch ohne Temperaturerhöhung unter Einstrahlung von UV-Licht erfolgen. Dies gilt insbesondere dann, wenn energiereiche Strahlung verwendet wird.

Bevorzugte Anwendung findet das erfindungsgemäße strahlungsempfindliche Gemisch in lithographischen Prozessen zur Herstellung integrierter Schaltungen oder von diskreten elektrischen Bausteinen. Das aus dem Gemisch hergestellte Aufzeichnungsmaterial dient dabei als Maske für die folgenden Prozeßschritte. Hierunter zählen z.B. das Ätzen des Schichtträgers, die Implantation von Ionen in den Schichtträger oder die Abscheidung von Metallen oder anderen Materialien auf den Schichtträger. Daneben ist das erfindungsgemäße strahlungsempfindliche Gemisch auch für die Herstellung von Druckplatten geeignet.

Die folgenden Beispiele erläutern die Herstellung der Monomeren, Homo- und Copolymeren sowie deren physikalische und lithographische Charakterisierung:

Die in den Beispielen angegebenen Teile und Prozente sind, soweit nicht anders angegeben, Gewichtsteile (Gt) bzw. Gewichtsprozente (Gew.-%).

Die Herstellung der N-(Hydroxyphenyl)-(meth)acrylamide erfolgt analog dem in "Sololawa et al., J.Gen.Chem. USSR (Engl.Transl.), 33, (1963), 1466" angegebenen Verfahren.

### Herstellungsbeispiel 1: N-(3-Hydroxy-phenyl)-methacrylamid

100 g 3-Amino-phenol werden in 500 ml Wasser suspendiert. Bei 50°C werden 154 g Methacrylsäureanhydrid zugetropft. Nach beendeter Zugabe wird noch 2h bei 70°C gerührt. Es fallen weiße Kristalle aus der wäßrigen Reaktionslösung aus. Man kühlt den Ansatz im Eisbad und saugt den Kristallkuchen ab, wäscht mit Wasser, trocknet und kristallisiert aus Acetonitril um.
Ausbeute: 90%, Schmelzpunkt: 176 °C

### Herstellungsbeispiel 2: N-(4-tert.-Butoxycarbonyloxy-phenyl)-methacrylamid

Zu einer Lösung aus 20,0 g (0,112 mol) N-(4-Hydroxy-phenyl)-methacrylamid in Tetrahydrofuran (THF) werden 30 g K₂CO₃ gegeben. Unter Rühren wird dann bei Raumtemperatur eine Lösung von 27 g (0,124 mol) Pyrokohlensäure-di-tert.-butylester (Di-tert.-butyldicarbonat) in THF zugetropft. Nach einigen Stunden ist die Umsetzung vollständig. Man gießt das Reaktionsgemisch auf Eis, extrahiert mit Essigester, trocknet die organische Phase und zieht das Lösemittel ab. Man erhält ein weißes kristallines Produkt, das aus Diisopropylether umkristallisiert wird.
Schmelzpunkt: 100 °C , Ausbeute: 95 %.

### Herstellungsbeispiel 3: N-(3-tert.-Butoxycarbonyloxy-phenyl)-methacrylamid

Zu einer Lösung aus 40,0 g (0,225 mol) N-(3-Hydroxy-phenyl)-methacrylamid in Tetrahydrofuran (THF) werden 50 g K₂CO₃ gegeben. Unter Rühren wird dann bei Raumtemperatur eine Lösung von 54,2 g (0,249 mol) Pyrokohlensäure-di-tert.-butylester (Di-tert.-butyldicarbonat) in THF zugetropft. Nach einigen Stunden ist die Umsetzung vollständig. Man gießt das Reaktionsgemisch auf Eis, extrahiert mit Essigester, trocknet die organische Phase und zieht das Lösemittel ab. Man erhält ein weißes kristallines Produkt, das aus Diisopropylether umkristallisiert wird.
Schmelzpunkt: 102 °C , Ausbeute: 95 %.

### Herstellungsbeispiel 4: N-(2-tert.-Butoxycarbonyloxy-phenyl)-methacrylamid

Zu einer Lösung aus 20,0 g (0,112 mol) N-(2-Hydroxy-phenyl)-methacrylamid in Tetrahydrofuran (THF) werden 30 g K₂CO₃ gegeben. Unter Rühren wird dann bei Raumtemperatur eine Lösung von 27 g (0,124 mol) Pyrokohlensäure-di-tert.-butylester (Di-tert.-Butyldicarbonat) in THF zugetropft. Nach einigen Stunden ist die Umsetzung vollständig. Man gießt das Reaktionsgemisch auf Eis, extrahiert mit Essigester, trocknet die organische Phase und zieht das Lösemittel ab. Man erhält ein gelbliches öl, das sich bei der Destillation zersetzt. Das Produkt wird ohne weitere Reinigung zur Polymerisation verwendet. Ausbeute: 95 %.

### Herstellungsbeispiele 5 bis 7: Poly[N-(2-hydroxy-phenyl)-methacrylamid] Poly[N-(3-hydroxy-phenyl)-methacrylamid] Poly[N-(4-hydroxy-phenyl)-methacrylamid]

10 g (56 mmol) des jeweiligen Monomers werden mit 0,370 g (2,3 mmol) Azobisisobutyronitril (AIBN) in 100 ml destilliertem THF 8 h unter Stickstoffatmosphäre zum Rückfluß erhitzt. Das Polymer wird in Petrolether ausgefällt und im Vakuumtrockenschrank bei 50 °C getrocknet.
Ausbeuten: 9-10 g

### Herstellungsbeispiele 8 bis 10: Poly[N-(2-tert.-butoxycarbonyloxy-phenyl)-methacrylamid] Poly[N-(3-tert.-butoxycarbonyloxy-phenyl)-methacrylamid] Poly[N-(4-tert.-butoxycarbonyloxy-phenyl)-methacrylamid]

15 g (54 mmol) des jeweiligen Monomers werden mit 0,355 g (2,1 mmol) Azobisisobutyronitril (AIBN) in 100 ml destilliertem THF 8 h unter Stickstoffatmosphäre zum Rückfluß erhitzt. Das Polymer wird in Petrolether ausgefällt und im Vakuumtrockenschrank bei 50 °C getrocknet.

Ausbeuten: 10-13 g
Molekulargewichte: M_{w}: 20000 g/mol, Mₙ: 12000g/mol

### Herstellungsbeispiel 11:

Copolymere aus N-(3-tert.-Butoxycarbonyloxy-phenyl)-methacrylamid und N-(3-Hydroxy-phenyl)-methacrylamid werden in verschiedenen Monomerverhältnissen unter den im Beispiel 6 angegebenen Bedingungen hergestellt. 20 mol% freier OH-Gruppen sind ausreichend, um gute Haftungseigenschaften zu erreichen.

Die folgenden Anwendungsbeispiele 1 bis 4 belegen die Eignung des erfindungsgemäßen Gemischs für Aufzeichnungsmaterialien in der Mikrolithographie. Die in diesen Beispielen eingesetzten 1-Sulfonyloxy-2-pyridone sowie Verfahren zu deren Herstellung sind in der nicht vorveröffentlichten deutschen Patentanmeldung P 41 12 967.9 beschrieben.

### Anwendungsbeispiel 1:

Es wird eine Lösung eines strahlungsempfindlichen Gemisches hergestellt, enthaltend
2,5 Gt eines Copolymers aus 30 % N-(3-Hydroxy-phenyl)-methacrylamid und 70 % N-(3-tert.-Butoxycarbonyloxy-phenyl)-methacrylamid und
0,075 Gt 4-Methyl-6-styryl-1-trifluormethansulfonyloxy-2-pyridon und
7,5 Gt Propylenglykol-monomethylether-acetat.

1,5 ml dieser Lösung werden durch einen Filter mit einem Porendurchmesser von 0,2 µm filtriert und auf einen Siliciumwafer, der mit Hexamethyldisilazan als Haftvermittler überzogen ist, aufgetragen. Durch Rotation bei 4500 Upm für 45 s wird eine ca. 1 µm dicke homogene Schicht erzeugt. Der Wafer wird auf einer Heizplatte bei 100 °C 60 s lang getrocknet, anschließend mit einer bildmäßig strukturierten Testmaske in Kontakt gebracht und unter Verwendung einer Xenon-Quecksilberdampflampe und einem zwischengeschalteten Interferenzfilter mit Strahlung von 365 nm bestrahlt (70 mJ/cm²). Danach wird der Wafer 1 min bei 80 °C gehalten und mit einer 0,27 n wäßrigen Tetramethylammoniumhydroxid-Lösung 180 s entwickelt. Strukturen von 0,70 µm (line/space) werden aufgelöst.

### Anwendungsbeispiel 2:

Es wird eine Lösung eines strahlungsempfindlichen Gemisches hergestellt, enthaltend
2,5 Gt eines Copolymers aus 30 % (2-Hydroxy-phenyl)-methacrylat und 70 % N-(3-tert.-butoxycarbonyloxy-phenyl)-methacrylamid und
0,075 Gt 4-Methyl-6-styryl-1-trifluormethansulfonyloxy-2-pyridon und
7,5 Gt Propylenglykol-monomethylether-acetat.

1,5 ml dieser Lösung werden durch einen Filter mit einem Porendurchmesser von 0,2 µm filtriert und auf einen Siliciumwafer, der mit Hexamethyldisilazan als Haftvermittler überzogen ist, aufgetragen. Durch Rotation bei 4500 Upm für 45 s wird eine ca. 1 µm dicke homogene Schicht erzeugt. Der Wafer wird auf einer Heizplatte bei 100 °C 60 s lang getrocknet, anschließend mit einer bildmäßig strukturierten Testmaske in Kontakt gebracht und unter Verwendung einer Xenon-Quecksilberdampflampe und einem zwischengeschalteten Interferenzfilter mit Strahlung von 365 nm bestrahlt (70 mJ/cm²). Danach wird der Wafer 1 min bei 80 °C gehalten und mit einer 0,27 n wäßrigen Tetramethylammoniumhydroxid-Lösung 180 s entwickelt. Strukturen von 0,70 µm (line/space) werden aufgelöst.

### Anwendungsbeispiel 3:

Es wird eine Lösung eines strahlungsempfindlichen Gemisches hergestellt, enthaltend
2,5 Gt eines Copolymers aus 30 % Methacrylsäure und 70 % N-(3-tert.-Butoxycarbonyloxy-phenyl)-methacrylamid und
0,075 Gt 4-Methyl-6-styryl-1-trifluormethansulfonyloxy-2-pyridon und
7,5 Gt Propylenglykol-monomethylether-acetat.

1,5 ml dieser Lösung werden durch einen Filter mit einem Porendurchmesser von 0,2 µm filtriert und auf einen Siliciumwafer, der mit Hexamethyldisilazan als Haftvermittler überzogen ist, aufgetragen. Durch Rotation bei 4500 Upm für 45 s wird eine ca. 1 µm dicke homogene Schicht erzeugt. Der Wafer wird auf einer Heizplatte bei 100 °C 60 s lang getrocknet, anschließend mit einer bildmäßig strukturierten Testmaske in Kontakt gebracht und unter Verwendung einer Xenon-Quecksilberdampflampe und einem zwischengeschalteten Interferenzfilter mit Strahlung von 365 nm bestrahlt (70 mJ/cm²). Danach wird der Wafer 1 min bei 80 °C gehalten und mit einer 0,18 n wäßrigen Tetramethylammoniumhydroxid-Lösung 60 s entwickelt. Strukturen von 0,70 µm (line/space) werden aufgelöst.

### Anwendungsbeispiel 4:

Es wird eine Lösung eines strahlungsempfindlichen Gemisches hergestellt aus
2,5 Gt Copolymer aus 30 % Brenzkatechin-monomethacrylat und 70 % N-(3-tert.-Butoxycarbonyloxy-phenyl)-methycrylamid,
0,075 Gt 4-Methyl-6-styryl-1-trifluormethansulfonyloxy-2-pyridon und
7,5 Gt Propylenglykol-monomethylether-acetat.

Von dieser Lösung werden 1,5 ml durch einen Filter mit einem Porendurchmesser von 0,2 µm filtriert und auf einen Siliciumwafer, der mit Hexamethyldisilazan als Haftvermittler überzogen ist, aufgetragen. Durch Rotation bei 4500 Upm für 45 s wird eine ca. 1 µm dicke homogene Schicht erzeugt. Der Wafer wird auf einer Heizplatte bei 100 °C 60 s lang getrocknet, anschließend mit einer bildmäßig strukturierten Testmaske in Kontakt gebracht und unter Verwendung einer Xenon-Quecksilberdampflampe und eines Interferenzfilters mit Strahlung von 248 nm bestrahlt (70 mJ/cm²). Danach wird der Wafer 1 min bei 80 °C gehalten und mit Anisol 120 s entwickelt. Strukturen von 0,70 µm (line/space) werden aufgelöst.

## Patentansprüche

1. Verbindung der allgemeinen Formel II 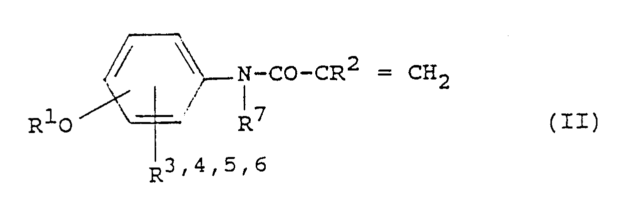 worin
R¹ eine Benzyl-, Trialkylsilanyl-, Alkoxycarbonyl-, Tetrahydropyranyl- oder Tetrahydrofuranyl-Gruppe
R² (C₁-C₄)Alkyl, Halogen(C₁-C₄)alkyl oder Wasserstoff bedeuten,
R^{3,4,5,6} voneinander unabhängig einen gegebenenfalls halogen-substituierten aliphatischen, araliphatischen oder aromatischen Rest mit 1 bis 20 Kohlenstoffatomen, ein Halogenatom oder Wasserstoff darstellen, und
R⁷ (C₁-C₄)Alkyl oder Wasserstoff ist.

2. Verbindung gemäß Anspruch 1, dadurch gekennzeichnet, daß
R² Wasserstoff oder eine Methylgruppe und
R³ bis R⁷ Wasserstoff sind.

3. Verbindung gemäß Anspruch 2, dadurch gekennzeichnet, daß die Gruppe R¹ eine tert.-Butoxycarbonyl-Gruppe ist und OR¹ sich in ortho-, meta- oder para-Stellung befindet.

4. Polymer mit Einheiten der allgemeinen Formel I 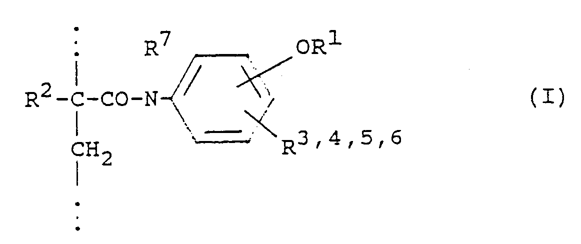 worin
R¹ eine Benzyl-, Trialkylsilyl-, Alkoxycarbonyl-, Tetrahydropyranyl- oder Tetrahydrofuranyl-Gruppe und
R² (C₁-C₄)Alkyl, Halogen(C₁-C₄)alkyl oder Wasserstoff bedeuten,
R^{3,4,5,6} voneinander unabhängig einen gegebenenfalls halogen-substituierten aliphatischen, araliphatischen oder aromatischen Rest mit 1 bis 20 Kohlenstoffatomen, ein Halogenatom oder Wasserstoff darstellen und
R⁷ (C₁-C₄)Alkyl oder Wasserstoff ist.

5. Polymer gemäß Anspruch 4, dadurch gekennzeichnet, daß
R² Wasserstoff oder eine Methylgruppe ist,
R³ bis R⁷ Wasserstoff sind.

6. Polymer gemäß Anspruch 4, dadurch gekennzeichnet, daß die Gruppe R¹ eine tert.-Butoxycarbonyl-Gruppe ist.

7. Polymer gemäß einem oder mehreren der Ansprüche 4 bis 6, dadurch gekennzeichnet, daß es zu mindestens 10 mol-% aus Einheiten der allgemeinen Formel II und zum Rest Einheiten, abgeleitet aus üblichen Vinylmonomeren, besteht.

8. Polymer gemäß einem oder mehreren der Ansprüche 4 bis 6, dadurch gekennzeichnet, daß es ein Homopolymer ist und neben den Einheiten der allgemeinen Formel I keine weiteren Einheiten enthält.

9. Polymer gemäß einem oder mehreren der Ansprüche 4 bis 6, dadurch gekennzeichnet, daß es ein Terpolymer ist und neben Einheiten der allgemeinen Formel I mit säurespaltbaren Gruppen Einheiten der allgemeinen Formel I mit R¹ = H oder einer nicht säurespaltbaren Gruppe und Einheiten, abgeleitet von einem weiteren polymerisierbaren Monomer, enthält.

10. Polymer gemäß einem oder mehreren der Ansprüche 4 bis 6, dadurch gekennzeichnet, daß es ein Terpolymer ist und neben Einheiten der allgemeinen Formel I mit säurespaltbaren Gruppen Einheiten der Formel I mit R¹ = H oder einer nicht säurespaltbaren Gruppe enthält.

11. Polymer gemäß einem oder mehreren der Ansprüche 4 bis 6, dadurch gekennzeichnet, daß es ein Copolymer ist und neben den Einheiten der allgemeinen Formel I mit säurespaltbaren Gruppen Einheiten, abgeleitet aus Maleinimid, enthält.

12. Polymer gemäß einem oder mehreren der Ansprüche 4 bis 6, dadurch gekennzeichnet, daß es ein Copolymer ist und neben Einheiten der allgemeinen Formel I mit säurespaltbaren Gruppen Einheiten, abgeleitet aus einem Ester oder Amid der (Meth)acrylsäure oder Methacrylsäure selber, enthält.

13. Polymer gemäß einem oder mehreren der Ansprüche 4 bis 6, dadurch gekennzeichnet, daß es ein Terpolymer ist und neben zwei verschiedenen Einheiten der allgemeinen Formel I mit säurespaltbaren Gruppen Einheiten abgeleitet aus einem Ester oder Amid der (Meth)acrylsäure oder Methacrylsäure selber, enthält.

14. Polymer gemäß einem oder mehreren der Ansprüche 4 bis 6, dadurch gekennzeichnet, daß es ein Copolymer ist und neben den Einheiten der allgemeinen Formel I mit säurespaltbaren Gruppen Einheiten, abgeleitet aus Vinylether, enthält.

15. Polymer gemäß einem oder mehreren der Ansprüche 4 bis 14, dadurch gekennzeichnet, daß es ein mittleres Molekulargewicht von 2.000 bis 100.000, bevorzugt 5.000 bis 40.000, aufweist.

16. Strahlungsempfindliches Gemisch mit einem polymeren Bindemittel, das durch Säure abspaltbare Seitengruppen aufweist, und einer bei Bestrahlung eine starke Säure bildende Verbindung, dadurch gekennzeichnet, daß das Bindemittel ein Polymer gemäß einem oder mehreren der Ansprüche 4 bis 15 umfaßt.

17. Strahlungsempfindliches Gemisch gemäß Anspruch 16, dadurch gekennzeichnet, daß der Säurebildner ein 1-Sulfonyloxy-2-pyridon ist.

18. Strahlungsempfindliches Gemisch gemäß Anspruch 16, dadurch gekennzeichnet, daß der Säurebildner ein Nitrobenzylester, ein Bissulfonyldiazomethan, ein Pyrogallolsulfonsäureester oder ein Oniumsalz ist.

19. Strahlungsempfindliches Gemisch gemäß einem oder mehreren der Ansprüche 16 bis 18, dadurch gekennzeichnet, daß der Anteil des Säurebildners im strahlungsempfindlichen Gemisch 1 bis 40 Gew.-%, insbesondere 3 bis 10 Gew.-%, bezogen auf das Gesamtgewicht der Feststoffe im Gemisch, beträgt.

20. Strahlungsempfindliches Gemisch gemäß einem oder mehreren der Ansprüche 16 bis 19, dadurch gekennzeichnet, daß der Anteil des Bindemittels im Gemisch 60 bis 99 Gew.-%, insbesondere 90 bis 97 Gew.-%, bezogen auf das Gesamtgewicht der Feststoffe im Gemisch, beträgt.

21. Aufzeichnungsmaterial mit einem Träger und einer darauf befindlichen strahlungsempfindlichen Schicht, dadurch gekennzeichnet, daß die strahlungsempfindliche Schicht ein strahlungsempfindliches Gemisch gemäß einem oder mehreren der Ansprüche 16 bis 20 enthält.

22. Verfahren zur Herstellung von Reliefstrukturen oder zur Strukturierung von Wafern durch Auftragen einer Photoresistschicht, die nach dem Trocknen eine Dicke von 0,1 bis 5 µm aufweist, auf ein in üblicher Weise vorbehandeltes Substrat, bildmäßiges Belichten, gegebenenfalls Erhitzen auf Temperaturen bis 150 °C und Entwickeln entweder mit einer wäßrig-alkalischen Lösung für ein positives Bild oder mit einem organischen Lösemittel für ein negatives Bild, dadurch gekennzeichnet, daß die Photoresistschicht ein strahlungsempfindliches Gemisch gemäß einem oder mehreren der Ansprüche 16 bis 20 enthält.

## Claims

1. A compound of the formula II 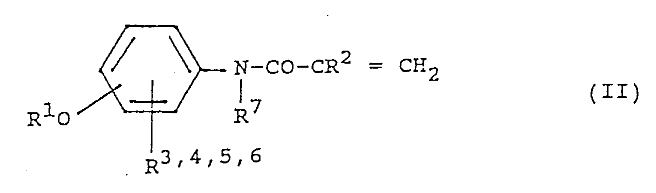 where
R¹ is a benzyl, trialkylsilanyl, alkoxycarbonyl, tetrahydropyranyl or tetrahydrofuranyl group,
R² is (C₁-C₄)alkyl, halogen(C₁-C₄)alkyl or hydrogen,
R^{3,4,5,6} are, independently of one another, an optionally halogen-substituted aliphatic, araliphatic or aromatic radical containing 1 to 20 carbon atoms, a halogen atom or hydrogen, and
R⁷ is (C₁-C₄)alkyl or hydrogen.

2. A compound as claimed in claim 1, wherein
R² is hydrogen or a methyl group,
R³ to R⁷ are hydrogen.

3. A compound as claimed in claim 2, wherein the group R¹ is a tert-butoxycarbonyl group and OR¹ is situated in the o-, m- or p-position.

4. A polymer embodying units of the formula I 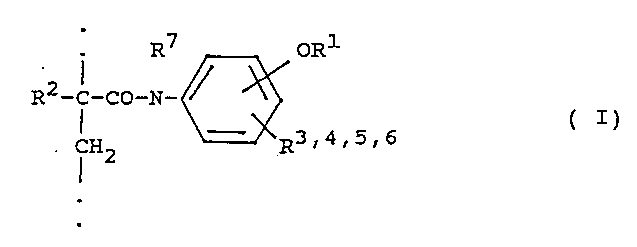 where
R¹ is a benzyl, trialkylsilanyl, alkoxycarbonyl, tetrahydropyranyl or tetrahydrofuranyl group,
R² is (C₁-C₄)alkyl, halogen(C₁-C₄)alkyl or hydrogen, and
R^{3,4,5,6} are, independently of one another, an optionally halogen-substituted aliphatic, araliphatic or aromatic radical containing 1 to 20 carbon atoms, a halogen atom or hydrogen,
R⁷ is (C₁-C₄)alkyl or hydrogen.

5. A polymer as claimed in claim 4, wherein
R² is hydrogen or a methyl group,
R³ to R⁷ are hydrogen.

6. A polymer as claimed in claim 4, wherein the group R¹ is a tert-butoxycarbonyl group.

7. A polymer as claimed in one or more of claims 4 to 6, which is composed of units of the formula II to an extent of at least 10 mol-% and, for the remainder, units derived from standard vinyl monomers.

8. A polymer as claimed in one or more of claims 4 to 6, which is a homopolymer and contains no further units in addition to the units of the formula I.

9. A polymer as claimed in one or more of claims 4 to 6, which is a terpolymer and contains, in addition to units of the formula I containing acid-cleavable groups, units of the formula I where R¹ = H or a non-acid-cleavable group and units derived from a further polymerizable monomer.

10. A polymer as claimed in one or more of claims 4 to 6, which is a terpolymer and contains, in addition to units of the formula I containing acid-cleavable groups, units of the formula I where R¹ = H or a non-acid-cleavable group.

11. A polymer as claimed in one or more of claims 4 to 6, which is a copolymer and contains, in addition to units of the formula I containing acid-cleavable groups, units derived from maleimide.

12. A polymer as claimed in one or more of claims 4 to 6, which is a copolymer and contains, in addition to units of the formula I containing acid-cleavable groups, units derived from an ester or amide of (meth)acrylic acid or methacrylic acid itself.

13. A polymer as claimed in one or more of claims 4 to 6, which is a terpolymer and contains, in addition to two different units of the formula I containing acid-cleavable groups, units derived from an ester or amide of (meth)acrylic acid or methacrylic acid itself.

14. A polymer as claimed in one or more of claims 4 to 6, which is a copolymer and contains, in addition to the units of the formula I containing acid-cleavable groups, units derived from vinyl ether.

15. A polymer as claimed in one or more of claims 4 to 14, which has an average molecular weight of 2,000 to 100,000, preferably 5,000 to 40,000.

16. A radiation-sensitive mixture containing a polymeric binder having acid-cleavable side groups, and a compound which forms a strong acid on irradiation, wherein the binder comprises a polymer as claimed in one or more of claims 4 to 15.

17. A radiation-sensitive mixture as claimed in claim 16, wherein the acid former is a 1-sulfonyloxy-2-pyridone.

18. A radiation-sensitive mixture as claimed in claim 16, wherein the acid former is a nitrobenzyl ester, a bissulfonyldiazomethane, a pyrogallosulfonate or an onium salt.

19. A radiation-sensitive mixture as claimed in one or more of claims 16 to 18, wherein the proportion of the acid former in the radiation-sensitive mixture is 1 to 40% by weight, in particular 3 to 10% by weight based on the total weight of solids in the mixture.

20. A radiation-sensitive mixture as claimed in one or more of claims 16 to 19, wherein the proportion of binder in the mixture is 60 to 99% by weight, in particular 90 to 97% by weight, based on the total weight of solids in the mixture.

21. A recording material comprising a base and a radiation-sensitive layer situated thereon, wherein the radiation-sensitive layer contains a radiation-sensitive mixture as claimed in one or more of claims 16 to 20.

22. A process for producing relief structures or for structuring wafers by applying a photoresist layer having a thickness of 0.1 to 5 µm after drying to a substrate pretreated in the standard way, imagewise exposing it, optionally heating it to temperatures of up to 150°C and developing it either with an agueous alkaline solution for a positive image or with an organic solvent for a negative image, wherein the photoresist layer contains a radiation-sensitive mixture as claimed in one or more of claims 16 to 20.

## Revendications

1. Composé de formule générale II : 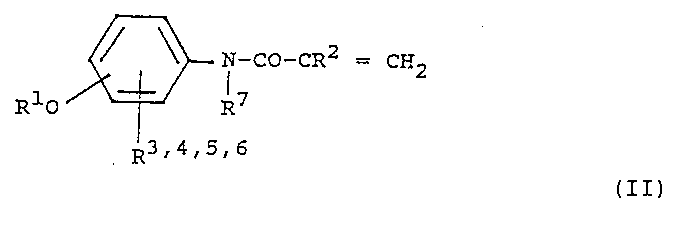 dans laquelle
R¹ représente un groupe benzyle, trialkylsilanyle, alkoxycarbonyle, tétrahydropyranyle ou tétrahydrofuranyle,
R² un groupe alkyle en C₁ à C₄, un halogénoalkyle en C₁ à C₄ ou un hydrogène,
R^{3,4,5,6} qui peuvent être identiques ou différents, représentent un reste aliphatique, araliphatique ou aromatique éventuellement substitué par un halogène, de 1 à 20 atomes de carbone, un atome d'halogène ou un hydrogène et
R⁷ est un groupe alkyle en C₁ à C₄ ou un hydrogène.

2. Composé selon la revendication 1, caractérisé en ce que
R² est un hydrogène ou un groupe méthyle et
R³ à R⁷ sont des hydrogènes.

3. Composé selon la revendication 2, caractérisé en ce que le groupe R¹ est un groupe tert.-butoxycarbonyle et OR¹ se trouve en position ortho, méta ou para.

4. Polymère composé de monomères de formule générale I 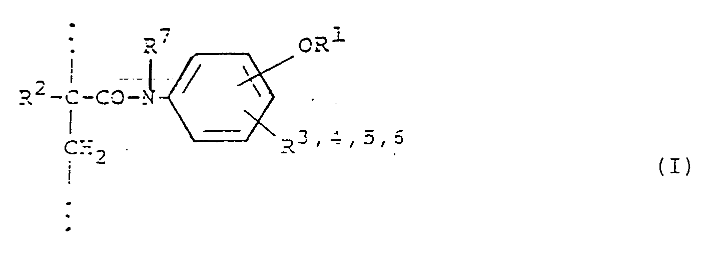 dans laquelle
R¹ représente un groupe benzyle, trialkylsilyle, alkoxycarbonyle, tétrahydropyranyle ou tétrahydrofunaryle et
R² un groupe alkyle en C₁ à C₄, un halogénoalkyle en C₁ à C₄ ou un hydrogène.
R^{3,4,5,6} qui peuvent être identiques ou différents, représentent un reste aliphatique, aralkyle ou aromatique éventuellement substitué par un halogène, de 1 à 20 atomes de carbone, un atome d'halogène ou un hydrogène et
R⁷ est un alkyle en C₁ à C₄ ou un hydrogène.

5. Polymère selon la revendication 4, caractérisé en ce que
R² est un hydrogène ou un groupe méthyle
R³ à R⁷ sont des hydrogènes.

6. Polymère selon la revendication 4, caractérisé en ce que le groupe R¹ est un groupe tert.-butoxycarbonyle.

7. Polymère selon une ou plusieurs des revendications 4 à 6, caractérisé en ce qu'au moins 10 mol-% sont de formule générale II, et le reste des unités dérive de monomères vinyliques usuels.

8. Polymère selon une ou plusieurs des revendications 4 à 6, caractérisé en ce qu'il est un homopolymère, qui ne contient pas à côté des unités de formule générale I, d'autres unités.

9. Polymère selon une ou plusieurs des revendications 4 à 6, caractérisé en ce qu'il est un terpolymère qui contient à côté d'unités de formule générale I avec des groupements clivables par les acides, des unités de formule générale I avec R¹=H ou un groupement non clivable par les acides, et des unités dérivées d'autres monomères polymérisables.

10. Polymère selon une ou plusieurs des revendications 4 à 6, caractérisé en ce qu'il' est un terpolymère, qui contient à côté d'unités de formule générale I avec des groupements clivables par les acides, des unités de formule générale I avec R¹=H ou un groupement non clivable par les acides.

11. Polymère selon une ou plusieurs des revendications 4 à 6, caractérisé en ce qu'il est un copolymère qui contient des unités de formule générale I avec des groupements clivables par les acides et des unités dérivées de maléinimide.

12. Polymère selon une ou plusieurs des revendications 4 à 6, caractérisé en ce qu'il est un copolymère, qui contient à côté d'unités de formule générale I avec des groupements clivables par les acides, des unités dérivées d'ester ou d'amide de l'acide (méth)acrylique ou de l'acide méthacrylique lui-même.

13. Polymère selon une ou plusieurs des revendications 4 à 6, caractérisé en ce qu'il est un terpolymère, qui contient à côté de deux unités différents de formule générale I avec des groupements clivables par les acides, des unités dérivées d'ester ou d'amide de l'acide (méth)acrylique ou de l'acide méthacrylique lui-même.

14. Polymère selon une ou plusieurs des revendications 4 à 6, caractérisé en ce qu'il est un copolymère qui contient à côté d'unités de formule générale I avec des groupements clivables par les acides, des unités dérivées d'éther vinylique.

15. Polymère selon une ou plusieurs des revendications 4 à 6, caractérisé en ce qu'il présente une masse moléculaire moyenne de 2.000 à 100.000, de préférence 5.000 à 40.000.

16. Composition sensible aux rayonnements contenant un polymère comme liant, qui présente une chaîne clivable par les acides et une chaîne qui sous irradiation produit un acide fort; caractérisée en ce que le liant comporte un polymère selon une ou plusieurs des revendications 4 à 15.

17. Composition sensible aux rayonnements selon la revendication 16, caractérisée en ce que le générateur d'acide est une 1-sulfonyloxy-2-pyridone.

18. Composition sensible aux rayonnements selon la revendication 16, caractérisée en ce que le générateur d'acide est un ester nitrobenzylique, un bis-sulfonyldiazométhane, un ester de l'acide pyrogallolsulfonique ou un sel d'onium.

19. Composition sensible aux rayonnements selon une ou plusieurs des revendications 16 à 18, caractérisée en ce que la proportion du générateur d'acide dans le mélange sensible aux rayonnements représente 1 à 40 % en poids plus particulièrement de 3 à 10 % en poids, rapporté au poids total des matières du mélange.

20. Composition sensible aux rayonnements selon une ou plusieurs revendications 16 à 19, caractérisée en ce que la proportion du liant dans le mélange représente 60 à 99 % en poids, plus particulièrement 90 à 97 % en poids, rapporté au poids total des matières du mélange.

21. Matériau de reprographie composé d'un support et d'une couche sensible aux rayonnements appliqués sur lui, caractérisée en ce que la couche sensible aux rayonnements contient une composition sensible aux rayonnements selon une ou plusieurs dès revendications 16 à 20.

22. Procédé pour obtenir des structures en relief ou pour structurer des pastilles par dépôt d'une couche de photorésist, qui après séchage présente une épaisseur de 0,1 à 5 µm, sur un substrat traité préalablement de manière usuelle, exposition à une image, éventullement chauffage à une température de 150°C et développement, soit par'une solution aqueuse alcaline pour obtenir une gage positive, soit par un solvant organique pour obtenir un négatif, caractérisé en ce que la couche de photorésist contient une composition sensible aux rayonnements selon une ou plusieurs des revendications 16 à 20.
